# EUROPEAN PATENT SPECIFICATION

(11) **EP 4 073 043 B1**
(45) Date of publication and mention of the grant of the patent: **03.04.2024**
(21) Application number: 20828093.3
(22) Date of filing: 11.12.2020
(51) Int. Cl.: C07D 239/36, A61P 11/00, C07D 239/91, A61K 31/513, A61K 31/517, A61K 31/5377

(54) **COMPOUNDS AND THEIR USE FOR THE TREATMENT OF ALPHA1-ANTITRYPSIN DEFICIENCY**
VERBINDUNGEN UND DEREN VERWENDUNG ZUR BEHANDLUNG VON ALPHA1-ANTITRYPSIN MANGEL
COMPOSÉS ET LEUR UTILISATION DANS LE TRAITEMENT D'UNE DÉFICIENCE EN ALPHA1-ANTITRYPSINE

(30) Priority: 13.12.2019 GB 201918404
(43) Date of publication of application: 19.10.2022
(73) Proprietor: Centessa Pharmaceuticals (UK) Limited, Altrincham, Cheshire WA14 2DT (GB)
(72) Inventor: RAMSDEN, Nigel, Babraham, Cambridge CB22 3FH (GB); FOX, David John, Coventry Warwickshire CV4 7AL (GB); HUNTINGTON, James Andrew, Altrincham, Cheshire WA14 2DT (GB); TOMLINSON, James Michael, Babraham, Cambridge CB22 3FH (GB)
(74) Representative: Roberts, Michael Austin
(86) International application number: PCT/GB2020/053191
(87) International publication number: WO 2021/116706

(56) References cited:
- WO-A1-2020/120992
- WO-A2-2008/143633
- DATABASE PubChem Compound [Online] NCBI; 28 January 2016 (2016-01-28), XP002801681, Database accession no. CID 113257346

## Description

The invention relates to certain benzamides and their medical use.

α₁-Antitrypsin (A1AT) is a member of the serpin superfamily produced by the liver and secreted into the blood. It inhibits a variety of serine proteases, especially neutrophil elastase. When blood levels of A1AT are low, excessive neutrophil elastase activity degrades lung tissue resulting in respiratory complications such as chronic obstructive pulmonary disease (COPD).

The reference range of A1AT in blood is 0.9-2.3 g/L. Levels lower than this are typical of α₁-antitrypsin deficiency (A1AD or AATD), a genetic disorder caused by mutations in the SERPINA1 gene, coding for A1AT. The Z mutation, the most common cause of AATD, is the substitution of glutamate to lysine at position 366 of A1AT (UniProtKB - P01009 (A1AT_HUMAN)), corresponding to position 342 in the mature protein (Z A1AT). The Z mutation affects the folding of A1AT resulting in only a small fraction acquiring the native/active state. The remainder is either cleared as misfolded protein or accumulates in the liver as stable polymers. As a consequence of the misfolding, homozygous carriers of the Z mutation (ZZ) have plasma levels of A1AT that are 10-15% of normal, predisposing carriers to COPD. Accumulation of Z A1AT polymers in liver cells predisposes carriers to cirrhosis, liver cancer and other liver pathologies.

The current treatment for the lung manifestation of AATD involves augmentation therapy using A1AT concentrates prepared from the plasma of blood donors. The US FDA has approved the use of four A1AT products: Prolastin, Zemaira, Glassia, and Aralast. Dosing is *via* once weekly intravenous infusion. Augmentation therapy has been demonstrated to slow progression of COPD. The liver manifestations of AATD (e.g. cirrhosis and cancer) are treated with steroids and liver transplantation. Investigational approaches to improved treatment of the liver manifestations include inhibition of Z A1AT polymerisation and increased clearance of polymers through the activation of autophagy. Investigational approaches to improved treatment of both the lung and the liver manifestations are directed towards improvement of Z A1AT folding and secretion.

Elliott et al (Protein Science, 2000, 9, 1274-1281) have described an X-ray crystal structure of A1AT and identified five cavities that are potential targets for rational drug design to develop agents that will affect Z A1AT polymerisation.

Parfrey et al (J. Biol. Chem., 2003, 278, 35, 33060-33066) have further defined a single cavity that is a potential target for rational drug design to develop agents that will affect Z A1AT polymerisation.

Knaupp et al (J. Mol. Biol., 2010, 396, 375-383) have shown that bis-ANS (4,4'-dianilino-1,1'-binaphthyl-5,5'-disulfonate) is able to bind to Z A1AT, but not to wild-type A1AT (M), with 1:1 stoichiometry and a K_{d} of 700nM.

Chang et al (J. Cell. Mol. Med., 2009, 13, 8B, 2304-2316) have reported a series of peptides, including Ac-TTAI-NH₂, that inhibit Z A1AT polymerization.

Burrows et al (Proc. Nat. Acad. Sci., 2000, 97, 4, 1796-1801) have shown that a series of non-selective chaperones, including 4-phenylbutyric acid, glycerol and trimethylamine oxide, are able to increase Z A1AT levels in cell supernatants and mouse models.

Bouchecareilh et al (Journal of Biological Chemistry, 2012, 287, 45, 38265-38278) describe the use of histone deacetylase inhibitors, in particular SAHA (suberoylanilide hydroxamic acid) to increase the secretion of both M and Z A1AT from cells.

Berthelier et al (PLOS ONE, May 11, 2015) have demonstrated that S-(4-nitrobenzyl)-6-thioguanosine is able to prevent Z A1AT polymerisation in vitro.

Mallya et al (J. Med. Chem., 2007, 50, 22, 5357-5363) describe a series of phenols, such as N-(4-hydroxy-3,5-dimethylphenyl)-2,5-dimethylthiophene-3-sulfonamide, able to block polymerisation of Z A1AT in vitro.

Huntington (XIIIth International Symposium on Proteinases, Inhibitors and Biological Control, 23 September 2012, and 7th International Symposium on Serpin Biology, Structure and Function, 1st April 2014) discussed a cavity from an X-ray crystal structure of Z A1AT that is a potential target for rational drug design to develop agents that will affect Z A1AT polymerisation.

US8,436,013B2 discloses a wide variety of structures able to increase secretion of Z A1AT from cells in the micromolar range.

Compounds with CAS registry numbers 1797054-78-4 and 1219580-65-0 are listed in the Aurora Building Blocks catalogue.

WO2019/243841A1 discloses oxoindoline-4-carboxamide compounds as modulators of alpha-1-antitrypsin, and use in treating diseases associated with alpha-1-antitrypsin.

WO2020/081257A1 discloses pyrrolo-indazolyl-propanoic acid compounds as modulators of alpha-1-antitrypsin.

US2020/0361939A1 discloses further pyrrolo-indazolyl-propanoic acid compounds as modulators of alpha-1-antitrypsin.

According to one aspect of the present invention, there is provided a compound of formula (1) 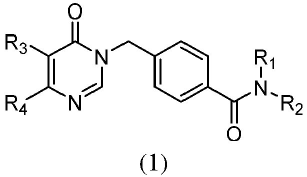 where:
- R₁, R₂, R₃ and R₄ are independently hydrogen or optionally substituted C₁-C₆ alkyl groups,
- R₁ and R₂ may be fused to form a heterocycle, and
- R₃ and R₄ may be fused to form a carbocycle
   and that when R₃ and R₄ are both hydrogen, R1 and R2 cannot
- both be methyl or perdeuterated methyl
- together be methyl and hydrogen wherein the compound of formula (1) is selected from
- N-Ethyl-N-methyl-4-((6-oxopyrimidin-1(6H)-yl)methyl)benzamide or
- 3-(4-(Pyrrolidine-1-carbonyl)benzyl)pyrimidin-4(3H)-one or
- 3-(4-(Morpholine-4-carbonyl)benzyl)pyrimidin-4(3H)-one or
- 3-(4-(4-Methylpiperazine-1-carbonyl)benzyl)pyrimidin-4(3H)-one or
- N-Methyl-4-((4-oxoquinazolin-3(4H)-yl)methyl)benzamide or
- 4-((6-Chloro-4-oxoquinazolin-3(4H)-yl)methyl)-N-methylbenzamide or
- N-isopropyl-N-methyl-4-((6-oxopyrimidin-1(6H)-yl)methyl)benzamide or
- N-benzyl-N-methyl-4-((6-oxopyrimidin-1(6H)-yl)methyl)benzamide or
- N-methyl-4-((6-oxopyrimidin-1(6H)-yl)methyl)-N-(2,2,2-trifluoroethyl)benzamide or
-
- N,N-diethyl-4-((6-oxopyrimidin-1(6H)-yl)methyl)benzamide or
- N-isopropyl-4-((6-oxopyrimidin-1(6H)-yl)methyl)benzamide or
- N-(4-fluorobenzyl)-4-((6-oxopyrimidin- 1(6H)-yl)methyl)benzamide or
- 4-((6-oxopyrimidin-1(6H)-yl)methyl)-N-(2,2,2-trifluoroethyl)benzamide or
- N-isopropyl-N-methyl-4-((4-oxoquinazolin-3(4H)-yl)methyl)benzamide or
- N-benzyl-N-methyl-4-((4-oxoquinazolin-3(4H)-yl)methyl)benzamide or
- N-methyl-4-((4-oxoquinazolin-3(4H)-yl)methyl)-N-(2,2,2-trifluoroethyl)benzamide or
- 4-((4,5-dimethyl-6-oxopyrimidin-1(6H)-yl)methyl)-N,N-dimethylbenzamide.

The compound of formula 1 cannot be
- N,N-dimethyl-4-((6-oxopyrimidin-1(6H)-yl)methyl)benzamide or
- N-methyl-4-((6-oxopyrimidin-1(6H)-yl)methyl)benzamide or
- N,N-bis(methyl-d₃)-4-((6-oxopyrimidin-1(6H)-yl)methyl)benzamide.

We have found that compounds of the invention are shown surprisingly to be highly effective at increasing levels of correctly folded, and hence active, Z A1AT, whilst having no effect on the secretion of wild type (M) A1AT or of the Siiyama variant of A1AT.

The compound of the invention may be in a pharmaceutically acceptable salt form or crystalline form.

The term "pharmaceutically acceptable salt" refers to a pharmaceutically acceptable mono organic or inorganic salt of the compound of the invention. This may include addition salts of inorganic acids such as hydrochloride, hydrobromide, hydroiodide, sulphate, phosphate, diphosphate and nitrate or of organic acids such as acetate, maleate, fumarate, tartrate, succinate, citrate, lactate, methanesulphonate, p-toluenesulphonate, palmoate and stearate. Exemplary salts also include oxalate, chloride, bromide, iodide, bisulphate, acid phosphate, isonicotinate, salicylate, acid citrate, oleate, tannate, pantothenate, bitartrate, ascorbate, gentisinate, gluconate, glucuronate, saccharate, formate, benzoate, glutamate, ethanesulfonate, and benzenesulfonate salts. For other examples of pharmaceutically acceptable salts, reference can be made to Gould (1986, Int J Pharm 33: 201-217).

According to a further aspect of the invention, there is a provided a pharmaceutical composition comprising the compound of the invention as described herein and a pharmaceutically or therapeutically acceptable excipient or carrier.

The term "pharmaceutically or therapeutically acceptable excipient or carrier" refers to a solid or liquid filler, diluent or encapsulating substance which does not interfere with the effectiveness or the biological activity of the active ingredients and which is not toxic to the host, which may be either humans or animals, to which it is administered. Depending upon the particular route of administration, a variety of pharmaceutically acceptable carriers such as those well known in the art may be used. Non-limiting examples include sugars, starches, cellulose and its derivatives, malt, gelatin, talc, calcium sulfate, vegetable oils, synthetic oils, polyols, alginic acid, phosphate buffered solutions, emulsifiers, isotonic saline, and pyrogen-free water.

All suitable modes of administration are contemplated according to the invention. For example, administration of the medicament may be via oral, subcutaneous, direct intravenous, slow intravenous infusion, continuous intravenous infusion, intravenous or epidural patient controlled analgesia (PCA and PCEA), intramuscular, intrathecal, epidural, intracistemal, intraperitoneal, transdermal, topical, transmucosal, buccal, sublingual, transmucosal, inhalation, intranasal, intra-atricular, intranasal, rectal or ocular routes. The medicament may be formulated in discrete dosage units and can be prepared by any of the methods well known in the art of pharmacy.

All suitable pharmaceutical dosage forms are contemplated. Administration of the medicament may for example be in the form of oral solutions and suspensions, tablets, capsules, lozenges, effervescent tablets, transmucosal films, suppositories, buccal products, oral mucoretentive products, topical creams, ointments, gels, films and patches, transdermal patches, abuse deterrent and abuse resistant formulations, sterile solutions suspensions and depots for parenteral use, and the like, administered as immediate release, sustained release, delayed release, controlled release, extended release and the like.

Another aspect of the invention is the use of the compound of the invention as defined herein in the manufacture of a medicament for the treatment of a disease or disorder.

A further aspect of the invention is the compound of the invention for use as an inducer of Z A1AT secretion.

Further provided is the compound of the invention as defined herein for use in the treatment of a disease or disorder.

Herein disclosed, but not claimed is a method of treating a disease or disorder, comprising the step of administering the compound or the pharmaceutical composition of the invention as defined herein to a patient in need of same.

Also disclosed, but not claimed is the use of a compound of the invention as an inducer of Z A1AT secretion. The use may be in the treatment of a disease or disorder. Additionally or alternatively, the use may be *in vitro*, for example in an *in vitro* assay. A disease or disorder suitable for treatment according to the relevant aspects of the invention is one which is characterised by low plasma levels of A1AT, for example AATD.

The use of a numerical range in this description is intended unambiguously to include within the scope of the invention all individual integers within the range and all the combinations of upper and lower limit numbers within the broadest scope of the given range.

As used herein, the term "comprising" is to be read as meaning both comprising and consisting of. Consequently, where the invention relates to a "pharmaceutical composition comprising as active ingredient" a compound, this terminology is intended to cover both compositions in which other active ingredients may be present and also compositions which consist only of one active ingredient as defined.

Unless otherwise defined, all the technical and scientific terms used here have the same meaning as that usually understood by an ordinary specialist in the field to which this invention belongs.

Particular non-limiting examples of the present invention will now be described.

### Experimental

### Example 1: N-Ethyl-N-methyl-4-((6-oxopyrimidin-1(6H)-yl)methyl)benzamide

N-Ethyl-N-methyl-4-((6-oxopyrimidin-1(6H)-yl)methyl)benzamide was prepared using the following sequential synthesis procedures.

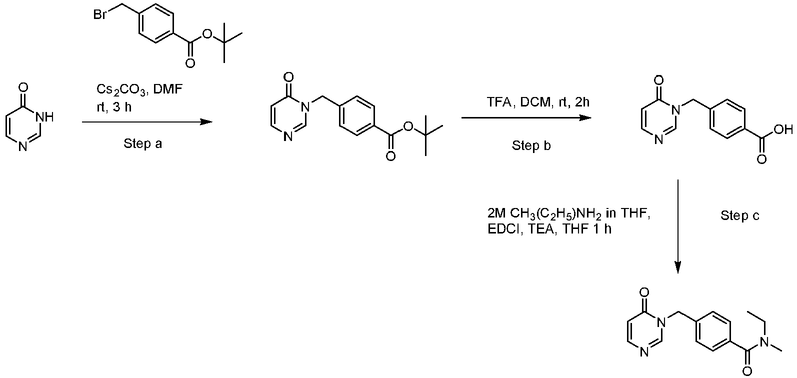

### Step a - Synthesis of tert-butyl 4-((6-oxopyrimidin-1(6H)-yl)methyl)benzoate

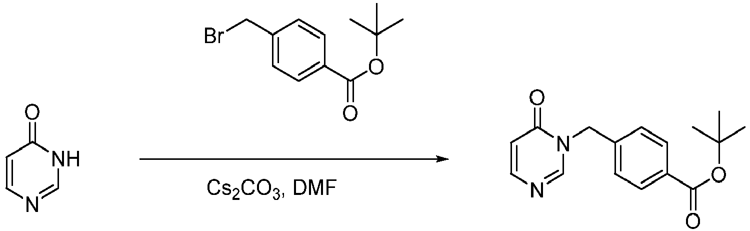

Pyrimidin-4(3H)-one (5g, 32 mmol) and caesium carbonate (50.85g, 156 mmol) were stirred in dimethylformamide (50ml) for 10 minutes at room temperature. Tert-butyl 4-(bromomethyl)benzoate (14.11g, 52 mmol) was added and the reaction was stirred for 3 hours. The reaction was diluted with water and the resulting yellow precipitate collected by filtration. The crude product was purified by column chromatography on silica, eluting with ethyl acetate/hexane (30 % to 33%) to give tert-butyl 4-((6-oxopyrimidin-1(6H)-yl)methyl)benzoate. Tlc Rf 0.2 1:1 Ethyl acetate/hexane.

### Step b - Synthesis of 4-((6-oxopyrimidin-1(6H)-yl)methyl)benzoic acid

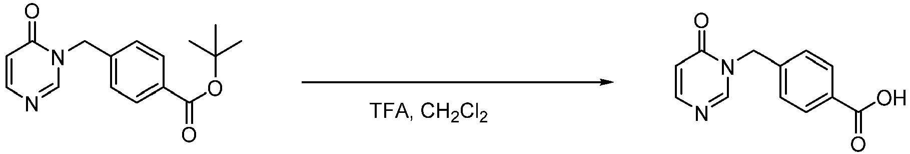

*Tert*-butyl 4-((6-oxopyrimidin-1(6H)-yl)methyl)benzoate (10g, 35 mmol) was dissolved in dichloromethane (50ml) and trifluoroacetic acid (70ml) was added slowly. The reaction was stirred for 3 hours at room temperature. The reaction was concentrated under reduced pressure and the resulting oil stirred with diethyl ether (300ml) for 20 minutes at room temperature. The resultant solid was collected by filtration, washed with diethyl ether (2 x 30ml) and dried in vacuo to give 4-((6-oxopyrimidin-1(6H)-yl)methyl)benzoic acid.

### Step c - Synthesis of N-ethyl-N-methyl-4-((6-oxopyrimidin-1(6H)-yl)methyl)benzamide

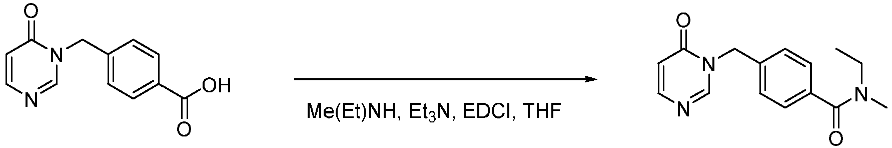

4-((6-oxopyrimidin-1(6H)-yl)methyl)benzoic acid (64mg, 0.27 mmol) and N-(3-dimethylaminopropyl)-N'-ethylcarbodiimide hydrochloride (86mg, 0.54 mmol) were stirred in tetrahydrofuran (1ml) for 10 minutes at 0°C under nitrogen. The reaction was then allowed to warm to room temperature. Triethylamine (0.11ml, 81 mmol) and ethylmethylamine (2M solution in tetrahydrofuran, 69 mmol) were added and the reaction was stirred for 2 hours. The reaction was concentrated under reduced pressure and the residue columned on silica eluting with 4% methanol in dichloromethane. Product containing fractions were concentrated to give N-ethyl-N-methyl-4-((6-oxopyrimidin-1(6H)-yl)methyl)benzamide.
m/z: 270.96 (calc 271.13)
¹H NMR (400 MHz, d6 DMSO) δ 8.69 (1H, s), 7.94 (1H, d), 7.36 (4H, s), 6.44 (1H, d), 5.13 (2H, s), 3.43 (1H, br s), 3.17 (1H, br s), 2.88 (3H, br s), 1.05 (3H, br s).

### Example 2: 3-(4-(Pyrrolidine-1-carbonyl)benzyl)pyrimidin-4(3H)-one

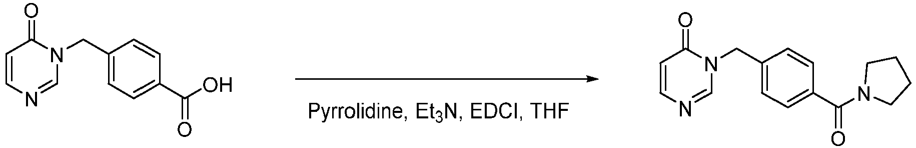

3-(4-(Pyrrolidine-1-carbonyl)benzyl)pyrimidin-4(3H)-one was prepared similarly to N-ethyl-N-methyl-4-((6-oxopyrimidin-1(6H)-yl)methyl)benzamide using pyrrolidine instead of ethylmethylamine in step c.
m/z: 282.91 (calc 283.13)
1H NMR (400 MHz, d6 DMSO) δ 8.69 (1H, s), 7.94 (1H, d), 7.49 (2H, d), 7.35 (2H, d), 6.44 (1H, d), 5.13 (2H, s), 3.44 (2H, t), 3.34 (2H, t), 1.83 (4H, m).

### Example 3: 3-(4-(Morpholine-4-carbonyl)benzyl)pyrimidin-4(3H)-one

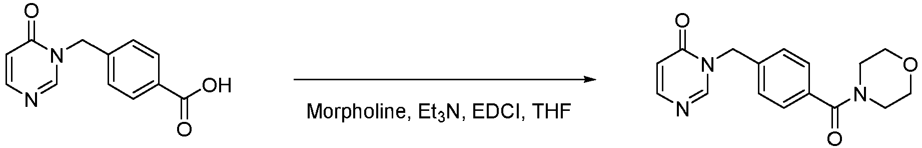

3-(4-(Morpholine-4-carbonyl)benzyl)pyrimidin-4(3H)-one was prepared similarly to N-ethyl-N-methyl-4-((6-oxopyrimidin-1(6H)-yl)methyl)benzamide using morpholine instead of ethylmethylamine in step c.
m/z: 298.97 (calc 299.13)
1H NMR (400 MHz, d6 DMSO) δ 8.69 (1H, s), 7.94 (1H, d), 7.38 (4H, m), 6.43 (1H, d), 5.13 (2H, s), 3.59-3.34 (8H, br m).

### Example 4: 3-(4-(4-Methylpiperazine-1-carbonyl)benzyl)pyrimidin-4(3H)-one

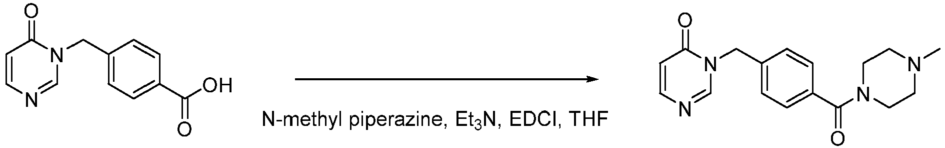

3-(4-(4-Methylpiperazine-1-carbonyl)benzyl)pyrimidin-4(3H)-one was prepared similarly to N-ethyl-N-methyl-4-((6-oxopyrimidin-1(6H)-yl)methyl)benzamide using N-methylpiperazine instead of ethylmethylamine in step c.
m/z: 312.18 (calc 312.16)
1H NMR (400 MHz, d6 DMSO) δ 8.69 (1H, s), 7.94 (1H, d), 7.36 (4H, s), 6.43 (1H, d), 5.13 (2H, s), 3.58 (2H, br), 3.33 (2H), 2.29 (4H, br m), 2.17 (3H, br s).

### Example 5: N-Methyl-4-((4-oxoquinazolin-3(4H)-yl)methyl)benzamide

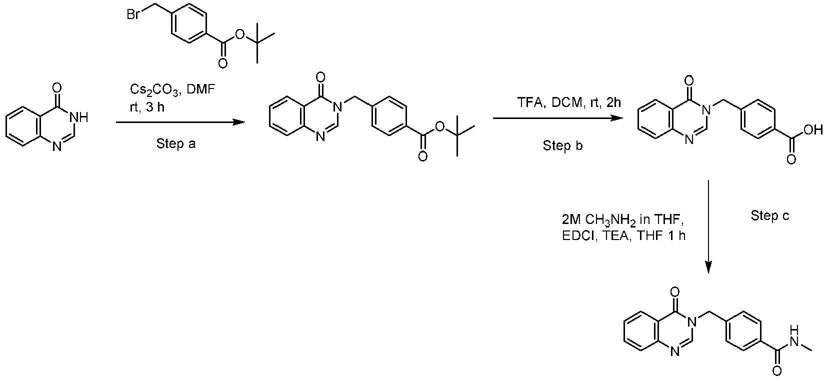

N-methyl-4-((4-oxoquinazolin-3(4H)-yl)methyl)benzamide was prepared similarly to N-ethyl-N-methyl-4-((6-oxopyrimidin-1(6H)-yl)methyl)benzamide using quinazolin-4(3H)-one instead of pyrimidin-4(3H)-one in step a and methylamine instead of ethylmethylamine in step c.
m/z: 292.92 (calc 293.12)
1H NMR (400 MHz, d6 DMSO) δ 8.60 (1H, s), 8.41 (1H, br m), 8.15 (1H, dd), 7.85 (1H, m), 7.79 (2H, d), 7.71 (1H, d), 7.56 (1H, m), 7.42 (2H, d), 5.24 (2H, s), 2.75 (3H, d).

### Example 6: 4-((6-chloro-4-oxoquinazolin-3(4H)-yl)methyl)-N-methylbenzamide

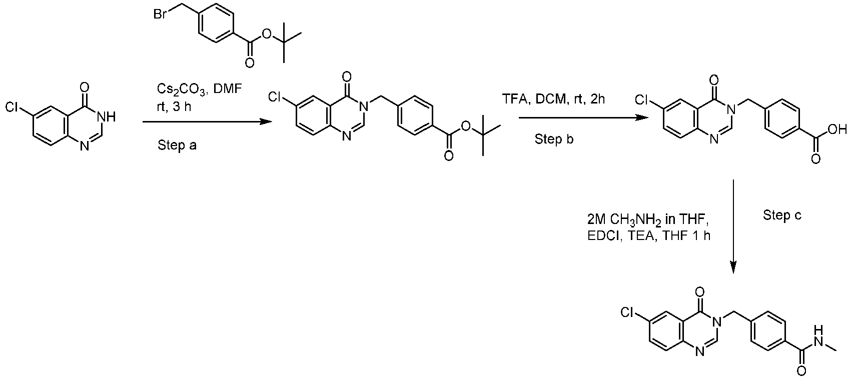

4-((6-chloro-4-oxoquinazolin-3(4H)-yl)methyl)-N-methylbenzamide was prepared similarly to N-methyl-4-((4-oxoquinazolin-3(4H)-yl)methyl)benzamide using 6-chloroquinazolin-4(3H)-one instead of quinazolin-4(3H)-one in step a.
m/z: 326.92, 329.10 (calc 327.08, 329.07)
1H NMR (400 MHz, d6 DMSO) δ 8.63 (1H, s), 8.42 (1H, br m), 8.09 (1H, d), 7.88 (1H, m), 7.79 (2H, d), 7.74 (1H, d), 7.42 (2H, d), 5.24 (2H, s), 2.75 (3H, d).

### Example 7: N-isopropyl-N-methyl-4-((6-oxopyrimidin-1(6H)-yl)methyl)benzamide

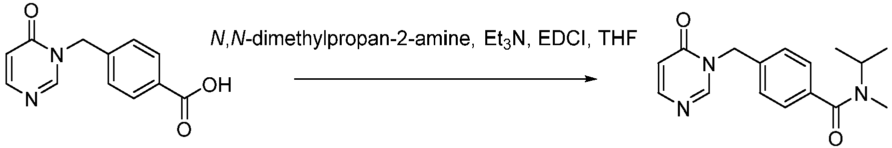

N-methyl-4-((6-oxopyrimidin-1(6H)-yl)methyl)benzamide was prepared similarly to N-ethyl-N-methyl-4-((6-oxopyrimidin-1(6H)-yl)methyl)benzamide using N,N-dimethylpropan-2-amine instead of ethylmethylamine in step c.
m/z: 285.10 (calc 285.15)
1H NMR (400 MHz, d6 DMSO) δ 8.68 (1H, s), 7.93 (1H, br m), 7.34 (4H, s), 6.43 (1H, br m), 5.12 (2H, s), 3.75 (1H br m), 2.67-2.74 (3H), 1.10 (6H).

### Example 8: N-benzyl-N-methyl-4-((6-oxopyrimidin-1(6H)-yl)methyl)benzamide

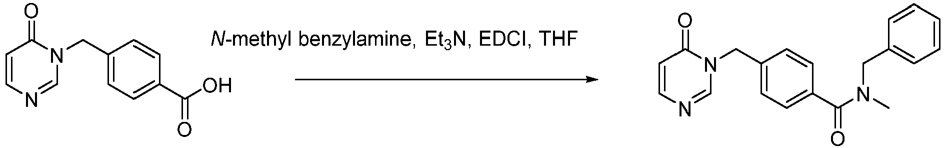

N-benzyl-N-methyl-4-((6-oxopyrimidin-1(6H)-yl)methyl)benzamidewas prepared similarly to N-ethyl-N-methyl-4-((6-oxopyrimidin-1(6H)-yl)methyl)benzamide using N-methyl benzylamine instead of ethylmethylamine in step c.
m/z: 333.13 (calc 333.15)
1H NMR (400 MHz, d6 DMSO) δ 8.60 (1H, s), 7.93 (1H, d), 7.41 (7H, m), 7.29 (3H, m), 6.42 (1H, d), 5.16 (2H, s).

### Example 9: N-methyl-4-((6-oxopyrimidin-1(6H)-yl)methyl)-N-(2,2,2-trifluoroethyl)benzamide.

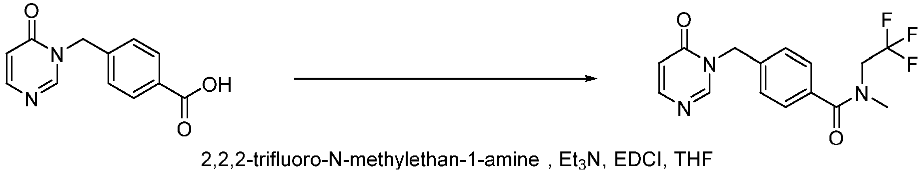

N-methyl-4-((6-oxopyrimidin-1(6H)-yl)methyl)-N-(2,2,2-trifluoroethyl)benzamide was prepared similarly to N-ethyl-N-methyl-4-((6-oxopyrimidin-1(6H)-yl)methyl)benzamide using 2,2,2-trifluoro-N-methylethan-1-amine instead of ethylmethylamine in step c.
m/z: 325.12 (calc 325.10)
1H NMR (400 MHz, d6 DMSO) δ 8.68 (1H, s), 7.94 (1H, d), 7.39 (4H, br s), 6.44 (1H, d), 5.13 (2H, s), 4.33 (2H, br), 3.00 (3H, br s).

### Example 10 : 4-((6-oxopyrimidin-1(6H)-yl)methyl)benzamide. (Reference example)

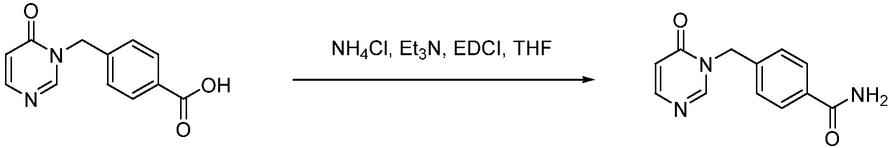

4-((6-oxopyrimidin-1(6H)-yl)methyl)benzamide was prepared similarly to N-ethyl-N-methyl-4-((6-oxopyrimidin-1(6H)-yl)methyl)benzamide using ammonium chloride instead of ethylmethylamine in step c.
m/z (M+Na): 252.07 (calc 252.07)
1H NMR (400 MHz, d6 DMSO) δ 8.68 (1H, s), 7.95 (1H, s,), 7.94 (1H, d), 7.86-7.81 (2H, m), 7.37 (1H, s), 7.40-7.34 (2H, m), 6.43 (1H, d), 5.14 (2H, s).

### Example 11: N,N-diethyl-4-((6-oxopyrimidin-1(6H)-yl)methyl)benzamide.

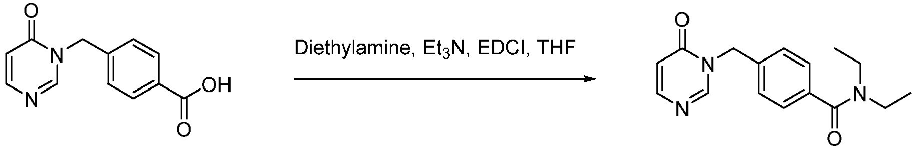

N,N-diethyl-4-((6-oxopyrimidin-1(6H)-yl)methyl)benzamide was prepared similarly to N-ethyl-N-methyl-4-((6-oxopyrimidin-1(6H)-yl)methyl)benzamide using diethylamine instead of ethylmethylamine in step c.
m/z (M+Na): 308.14 (calc 308.14)
1H NMR (400 MHz, d6 DMSO) δ 8.68 (1H, s), 7.94 (1H, d), 7.37-7.30 (4H, m), 6.44 (1H, d), 5.13 (2H, s), 3.48-3.37 (2H, m), 3.22-3.08 (2H, m), 1.17-1.08 (3H, m), 1.08-0.96 (3H, m).

### Example 12: N-isopropyl-4-((6-oxopyrimidin-1(6H)-yl)methyl)benzamide.

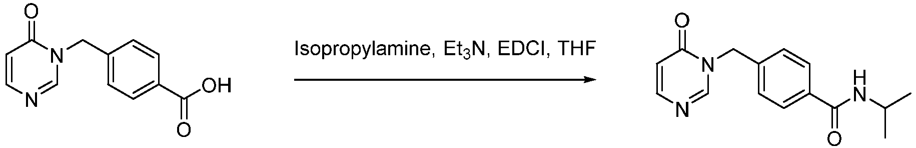

N-isopropyl-4-((6-oxopyrimidin-1(6H)-yl)methyl)benzamide was prepared similarly to N-ethyl-N-methyl-4-((6-oxopyrimidin-1(6H)-yl)methyl)benzamide using isopropylamine instead of ethylmethylamine in step c.
m/z (M+Na): 294.12 (calc 294.12)
1H NMR (400 MHz, d6 DMSO) δ 8.63 (1H, s), 7.93 (1H, s), 7.79-7.73 (2H, m), 7.38-7.32 (2H, m), 6.43 (1H, d), 5.12 (2H, s), 4.04 (1H, sept.), 1.12 (6H, d).

### Example 13: N-(4-fluorobenzyl)-4-((6-oxopyrimidin-1(6H)-yl)methyl)benzamide.

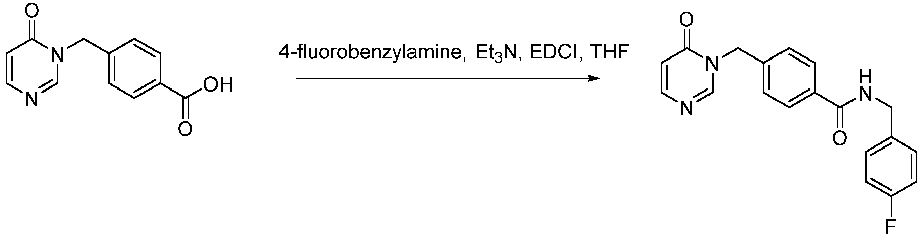

N-(4-fluorobenzyl)-4-((6-oxopyrimidin-1(6H)-yl)methyl)benzamide was prepared similarly to N-ethyl-N-methyl-4-((6-oxopyrimidin-1(6H)-yl)methyl)benzamide using 4-fluorobenzylamine instead of ethylmethylamine in step c.
m/z (M+Na): 360.11 (calc 360.11)
1H NMR (400 MHz, d6 DMSO) δ 9.03 (1H, t), 8.68 (1H, s), 7.94 (1H, d), 7.88-7.83 (2H, m), 7.42-7.38 (2H, m), 7.34 (2H, dd), 7.17-7.11 (2H, m), 6.43 (1H, d), 5.15 (2H, s), 4.44 (2H, d).

### Example 14: 4-((6-oxopyrimidin-1(6H)-yl)methyl)-N-(2,2,2-trifluoroethyl)benzamide.

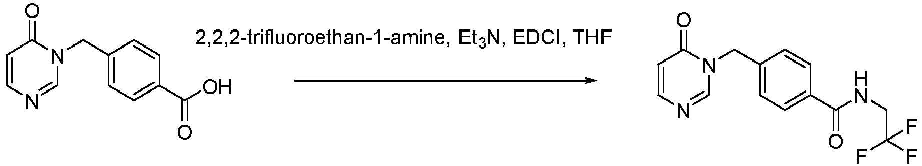

4-((6-oxopyrimidin-1(6H)-yl)methyl)-N-(2,2,2-trifluoroethyl)benzamide was prepared similarly to N-ethyl-N-methyl-4-((6-oxopyrimidin-1(6H)-yl)methyl)benzamide using 2,2,2-trifluoroethan-1-amine instead of ethylmethylamine in step c.
m/z (M+Na): 334.08 (calc 334.08)
1H NMR (400 MHz, d6 DMSO) δ 9.07 (1H, t), 8.69 (1H, s), 7.95 (1H, d), 7.89-7.81 (2H, m), 7.47-7.36 (2H, m), 6.44 (1H, d), 5.16 (2H, s), 4.08 (2H, qd).

### Example 15: N-isopropyl-N-methyl-4-((4-oxoquinazolin-3(4H)-yl)methyl)benzamide

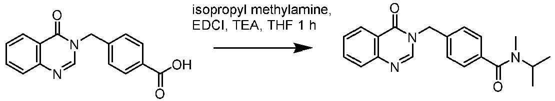

N-isopropyl-N-methyl-4-((4-oxoquinazolin-3(4H)-yl)methyl)benzamide was prepared similarly to N-Methyl-4-((4-oxoquinazolin-3(4H)-yl)methyl)benzamide using isopropyl methylamine in step c.
m/z: 335.28 (calc 335.16)
1H NMR (400 MHz, d6 DMSO) δ 8.59 (1H, s), 8.15 (1H, d), 7.84 (1H, t), 7.70 (1H, d), 7.56 (1H, t), 7.39 (2H, m), 7.32 (2H, br), 5.22 (2H, s), 4.66 and 3.75 (1H, br), 2.86-2.66 (3H, br), 1.06 (6H, br).

### Example 16: N-benzyl-N-methyl-4-((4-oxoquinazolin-3(4H)-yl)methyl)benzamide

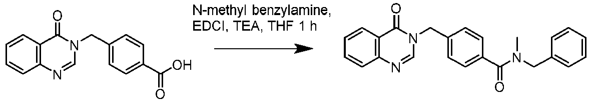

N-benzyl-N-methyl-4-((4-oxoquinazolin-3(4H)-yl)methyl)benzamide was prepared similarly to N-Methyl-4-((4-oxoquinazolin-3(4H)-yl)methyl)benzamide using N-methyl benzylamine in step c.
m/z: 383.19 (calc 383.16)
1H NMR (400 MHz, d6 DMSO) δ 8.58 (1H, d), 8.14 (1H, d), 7.83 (1H, t), 7.70 (1H, d), 7.75 (1H, t), 7.40-7.00 (9H, br), 5.23 (2H, s), 4.64 and 4.43 (2H, br), 2.84-2.78 (3H, br).

### Example 17: N-methyl-4-((4-oxoquinazolin-3(4H)-yl)methyl)-N-(2,2,2-trifluoroethyl)benzamide.

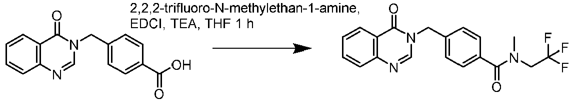

N-methyl-4-((4-oxoquinazolin-3(4H)-yl)methyl)-N-(2,2,2-trifluoroethyl)benzamide was prepared similarly to N-Methyl-4-((4-oxoquinazolin-3(4H)-yl)methyl)benzamide using 2,2,2-trifluoro-N-methylethan-1-amine in step c.
m/z: 375.19 (calc 375.12)
1H NMR (400 MHz, d6 DMSO) δ 8.56 (1H, d), 8.12 (1H, d), 7.81 (1H, t), 7.67 (1H, d), 7.52 (1H, t), 7.39 (4H, br), 5.21 (2H, s), 4.29 and 4.10 (2H, br), 3.00 (3H, br).

### Example 18: 4-((4,5-dimethyl-6-oxopyrimidin-1(6H)-yl)methyl)-N,N-dimethylbenzamide.

4-((4,5-dimethyl-6-oxopyrimidin-1(6H)-yl)methyl)-N,N-dimethylbenzamide was prepared similarly to N-ethyl-N-methyl-4-((6-oxopyrimidin-1(6H)-yl)methyl)benzamide using 5,6-dimethylpyrimidin-4(3H)-one instead of pyrimidin-4(3H)-one in step a and dimethylamine instead of ethylmethylamine in step c.
m/z: 285.01 (calc 285.15)
1H NMR (400 MHz, d6 DMSO) δ 8.45 (1H, s), 7.34 (4H, ABq), 5.09 (2H, s), 3.95-2.86 (3H, br), 2.20 (3H, s), 1.93 (3H, s).

### Example 19: Activity of compounds of the invention in an A1AT cell secretion assay using HEK-Z cells

### Methods

HEK-Z cells, a human embryonic kidney cell line stably transfected with the human Z A1AT gene, were plated into 96 well plates (3.0 x 10⁵ cells/ml with 200 µl of media/well) overnight at 37°C in a humidified atmosphere containing 5% CO₂. Following incubation cells were washed with 200 µl serum-free media three times and media was replaced with treatments in quadruplicate using serum free media containing either vehicle, 10 µM suberanilohydroxamic acid (SAHA) or a compound of the invention (at concentrations of 10, 33, 100 and 333 nM) for 48 h in a 37°C incubator in a final volume of 200 µl. At the end of the incubation step the supernatants were removed from the wells, centrifuged at 1000 *x* g at 4°C for 10 min and were assayed for human A1AT levels by ELISA (Human Serpin A1/α₁-antitrypsin duo set ELISA, R& D Systems, DY1268) per manufacturer's instructions.

Briefly, a 96 well plate was coated with human A1AT capture antibody overnight at room temperature (1:180 dilution from stock, 100 µl final volume/well). The capture antibody was then removed and wells washed three times with 300 µl wash buffer (0.05% Tween 20 in PBS) and then 200 µl reagent diluent (25% Tween 20 in PBS) was incubated in each well for 1 h at room temperature. Diluted samples, standards (125, 250, 500, 1000, 2000, 4000 and 8000 pg/ml A1AT) or blanks were then added to each well in duplicate and the plates were covered with a plate sealer and left at room temperature for 2 h. At the end of the sample incubation step, samples were removed and all wells washed as previously and 100 µl detection antibody (1:180 dilution from stock) was added to each well and incubated for a further 2 h at room temperature. Following incubation with detection antibody, supernatant was removed and wells were washed as previously and 100 µl streptavidin-HRP solution (1:200 dilution from stock) was added to each well for 20 min in the dark. After which, 50 µl stop solution (2M H₂SO₄) was added and optical density (OD) of each well was read at 450 nm with 570 nm blank subtracted from each well using a microplate reader. A 4 parameter logistic curve was constructed using GraphPad Prism 7 and A1AT concentrations were determined in each sample by interpolation from a standard curve and multiplying by the appropriate dilution factor.

### Results

The data in Table 1 show that compounds of Examples 1-18 increase secretion of Z A1AT from HEK-Z cells at 300nM.

**Table 1**

| Example | Median A1AT % increase over vehicle at 300nM |
|---|---|
| 1 | 330 |
| 2 | 350 |
| 3 | 280 |
| 4 | 390 |
| 5 | 180 |
| 6 | 200 |
| 7 | 160 |
| 8 | 150 |
| 9 | 170 |
| 10 | 260 |
| 11 | 230 |
| 12 | 210 |
| 13 | 190 |
| 14 | 180 |
| 15 | 190 |
| 16 | 160 |
| 17 | 170 |
| 18 | 170 |

## Claims

1. A compound of formula (1) 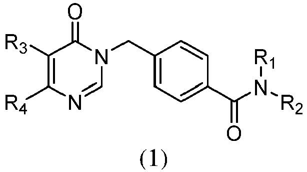 where
• R₁, R₂, R₃ and R₄ are independently hydrogen or optionally substituted C₁-C₆ alkyl groups,
• R₁ and R₂ may be fused to form a heterocycle,
• R₃ and R₄ may be fused to form a carbocycle,
and that when R₃ and R₄ are both hydrogen, R1 and R2 cannot
• both be methyl or perdeuterated methyl
• together be methyl and hydrogen wherein the compound of formula (1) is selected from
• N-Ethyl-N-methyl-4-((6-oxopyrimidin-1(6H)-yl)methyl)benzamide or
• 3-(4-(Pyrrolidine-1-carbonyl)benzyl)pyrimidin-4(3H)-one or
• 3-(4-(Morpholine-4-carbonyl)benzyl)pyrimidin-4(3H)-one or
• 3-(4-(4-Methylpiperazine-1-carbonyl)benzyl)pyrimidin-4(3H)-one or
• N-Methyl-4-((4-oxoquinazolin-3(4H)-yl)methyl)benzamide or
• 4-((6-Chloro-4-oxoquinazolin-3(4H)-yl)methyl)-N-methylbenzamide or
• N-Isopropyl-N-methyl-4-((6-oxopyrimidin-1(6H)-yl)methyl)benzamide or
• N-Benzyl-N-methyl-4-((6-oxopyrimidin-1(6H)-yl)methyl)benzamide or
• N-Methyl-4-((6-oxopyrimidin-1(6H)-yl)methyl)-N-(2,2,2-trifluoroethyl)benzamide or
• N,N-Diethyl-4-((6-oxopyrimidin-1(6H)-yl)methyl)benzamide or
• N-Isopropyl-4-((6-oxopyrimidin-1(6H)-yl)methyl)benzamide or
• N-(4-Fluorobenzyl)-4-((6-oxopyrimidin-1(6H)-yl)methyl)benzamide or
• 4-((6-Oxopyrimidin-1(6H)-yl)methyl)-N-(2,2,2-trifluoroethyl)benzamide or
• N-isopropyl-N-methyl-4-((4-oxoquinazolin-3(4H)-yl)methyl)benzamide or
• N-benzyl-N-methyl-4-((4-oxoquinazolin-3(4H)-yl)methyl)benzamide or
• N-methyl-4-((4-oxoquinazolin-3(4H)-yl)methyl)-N-(2,2,2-trifluoroethyl)benzamide or
• 4-((4,5-dimethyl-6-oxopyrimidin-1(6H)-yl)methyl)-N,N-dimethylbenzamide.

2. The compound according to claim 1 in a pharmaceutically acceptable salt form or crystalline form.

3. A pharmaceutical composition comprising a compound according to either of claims 1 or 2 and a pharmaceutically or therapeutically acceptable excipient or carrier.

4. A compound according to either of claims 1 or 2 for use in the treatment of a disease or disorder.

5. The compound for use according to claim 4, wherein the disease or disorder is α1-antitrypsin deficiency (AATD).

## Patentansprüche

1. Verbindung von Formel (1) 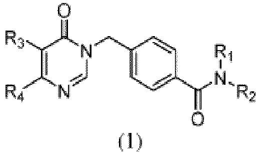 wobei
• R₁, R₂, R₃ und R₄ unabhängig voneinander Wasserstoff oder optional substituierte C₁-C₆-Alkylgruppen sind,
• R₁ und R₂ zu einem Heterocyclus kondensiert sein können,
• R₃ und R₄ zu einem Carbocyclus kondensiert sein können,
und wenn R₃ und R₄ beide Wasserstoff sind, R₁ und R₂ nicht
• beide Methyl oder perdeuteriertes Methyl sein können
• zusammen Methyl und Wasserstoff sein können wobei die Verbindung von Formel (1) ausgewählt ist aus
• N-Ethyl-N-methyl-4-((6-oxopyrimidin-1(6H)-yl)methyl)benzamid oder
• 3-(4-(Pyrrolidin-1-carbonyl)benzyl)pyrimidin-4(3H)-on oder
• 3-(4-(Morpholin-4-carbonyl)benzyl)pyrimidin-4(3H)-on oder
• 3-(4-(4-Methylpiperazin-1-carbonyl)benzyl)pyrimidin-4(3H)-on oder
• N-Methyl-4-((4-oxochinazolin-3(4H)-yl)methyl)benzamid oder
• 4-((6-Chloro-4-oxochinazolin-3(4H)-yl)methyl)-N-Methylbenzamid oder
• N-Isopropyl-N-methyl-4-((6-oxopyrimidin-1(6H)-yl)methyl)benzamid oder
• N-Benzyl-N-methyl-4-((6-oxopyrimidin-1(6H)-yl)methyl)benzamid oder
• N-Methyl-4-((6-oxopyrimidin-1(6H)-yl)methyl)-N-(2,2,2-trifluorethyl)benzamid oder
• N,N-Diethyl-4-((6-oxopyrimidin-1(6H)-yl)methyl)benzamid oder
• N-Isopropyl-4-((6-oxopyrimidin-1(6H)-yl)methyl)benzamid oder
• N-(4-Fluorbenzyl)-4-((6-oxopyrimidin-1(6H)-yl)methyl)benzamid oder
• 4-((6-Oxopyrimidin-1(6H)-yl)methyl)-N-(2,2,2-trifluorethyl)benzamid oder
• N-Isopropyl-N-methyl-4-((4-oxochinazolin-3(4H)-yl)methyl)benzamid oder
• N-Benzyl-N-methyl-4-((4-oxochinazolin-3(4H)-yl)methyl)benzamid oder
• N-methyl-4-((4-oxochinazolin-3(4H)-yl)methyl)-N-(2,2,2-trifluorethyl)benzamid oder
• 4-((4,5-Dimethyl-6-oxopyrimidin-1(6H)-yl)methyl)-N,N-dimethylbenzamid.

2. Verbindung nach Anspruch 1 in einer pharmazeutisch akzeptablen Salzform oder kristallinen Form.

3. Pharmazeutische Zusammensetzung, die eine Verbindung nach Anspruch 1 oder 2 und einen pharmazeutisch oder therapeutisch akzaptablen Hilfsstoff oder Träger umfasst.

4. Verbindung nach Anspruch 1 oder 2 zur Verwendung bei der Behandlung einer Krankheit oder Störung.

5. Verbindung zur Verwendung nach Anspruch 4, wobei die Krankheit oder Störung ein α1-Antitrypsinmangel (AATD) ist.

## Revendications

1. Composé de formule (1) 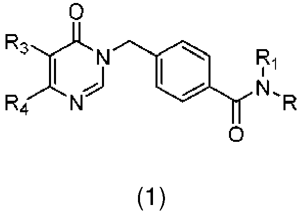 où
• R₁, R₂, R₃ et R₄ sont indépendamment hydrogène ou des groupes alkyle en C₁-C₆ facultativement substitués,
• R₁ et R₂ peuvent être fusionnés pour former un hétérocycle,
• R₃ et R₄ peuvent être fusionnés pour former un carbocycle,
et que lorsque R₃ et R₄ sont tous les deux hydrogène, R1 et R2 ne peuvent pas
• tous les deux être méthyle ou méthyle perdeutéré
• ensemble être méthyle et hydrogène
dans lequel le composé de formule (1) est choisi parmi
• N-éthyl-N-méthyl-4-((6-oxopyrimidin-1(6H)-yl)méthyl)benzamide ou
• 3-(4-(pyrrolidine-1-carbonyl)benzyl)pyrimidin-4(3H)-one ou
• 3-(4-(morpholine-4-carbonyl)benzyl)pyrimidin-4(3H)-one ou
• 3-(4-(4-méthylpipérazine-1-carbonyl)benzyl)pyrimidin-4(3H)-one ou
• N-méthyl-4-((4-oxoquinazolin-3(4H)-yl)méthyl)benzamide ou
• 4-((6-chloro-4-oxoquinazolin-3(4H)-yl)méthyl)-N-méthylbenzamide ou
• N-isopropyl-N-méthyl-4-((6-oxopyrimidin-1(6H)-yl)méthyl)benzamide ou
• N-benzyl-N-méthyl-4-((6-oxopyrimidin-1(6H)-yl)méthyl)benzamide ou
• N-méthyl-4-((6-oxopyrimidin-1(6H)-yl)méthyl)-N-(2,2,2-trifluoroéthyl)benzamide ou
• N,N-diéthyl-4-((6-oxopyrimidin-1(6H)-yl)méthyl)benzamide ou
• N-isopropyl-4-((6-oxopyrimidin-1(6H)-yl)méthyl)benzamide ou
• N-(4-fluorobenzyl)-4-((6-oxopyrimidin-1(6H)-yl)méthyl)benzamide ou
• 4-((6-oxopyrimidin-1(6H)-yl)méthyl)-N-(2,2,2-trifluoroéthyl)benzamide ou
• N-isopropyl-N-méthyl-4-((4-oxoquinazolin-3(4H)-yl)méthyl)benzamide ou
• N-benzyl-N-méthyl-4-((4-oxoquinazolin-3(4H)-yl)méthyl)benzamide ou
• N-méthyl-4-((4-oxoquinazolin-3(4H)-yl)méthyl)-N-(2,2,2-trifluoroéthyl)benzamide ou
• 4-((4,5-diméthyl-6-oxopyrimidin-1(6H)-yl)méthyl)-N,N-diméthylbenzamide.

2. Composé selon la revendication 1 sous une forme de sel ou forme cristalline pharmaceutiquement acceptable.

3. Composition pharmaceutique comprenant un composé selon l'une quelconque des revendications 1 ou 2 et un excipient ou véhicule pharmaceutiquement ou thérapeutiquement acceptable.

4. Composé selon l'une quelconque des revendications 1 ou 2 pour une utilisation dans le traitement d'une maladie ou d'un trouble.

5. Composé pour une utilisation selon la revendication 4, dans lequel la maladie ou le trouble est un déficit en α1-antitrypsine (AATD).
